# EUROPÄISCHE PATENTSCHRIFT

(11) **EP 2 916 743 B1**
(45) Veröffentlichungstag und Bekanntmachung des Hinweises auf die Patenterteilung: **06.12.2017**
(21) Anmeldenummer: 13783940.3
(22) Anmeldetag: 30.10.2013
(51) Int. Cl.: A61B 17/072, A61B 17/115, A61B 18/14

(54) **ELEKTROCHIRURGISCHES INSTRUMENT MIT KLEMMDRUCKREGELUNG FÜR ELEKTRODENBRANCHEN**
ELECTROSURGICAL INSTRUMENT WITH CLAMPING PRESSURE CONTROL FOR ELECTRODE BRANCHES
INSTRUMENT ÉLECTROCHIRURGICAL AVEC RÉGLAGE DE LA PRESSION DE PINCEMENT DE BRANCHES D'ÉLECTRODES

(30) Priorität: 07.11.2012 DE 102012110660
(43) Veröffentlichungstag der Anmeldung: 16.09.2015
(73) Patentinhaber: Aesculap AG, 78532 Tuttlingen (DE)
(72) Erfinder: ROTHWEILER, Christoph, 78166 Donaueschingen (DE); HAFNER, Nikolaus, 78532 Tuttlingen (DE); HERNER, Eugen, 78054 Villingen-Schwenningen (DE); HEIZMANN, Patrick, 78183 Hüfingen (DE)
(74) Vertreter: Winter, Brandl, Fürniss, Hübner, Röss, Kaiser, Polte - Partnerschaft mbB
(86) Internationale Anmeldenummer: PCT/EP2013/072721
(87) Internationale Veröffentlichungsnummer: WO 2014/072215

(56) Entgegenhaltungen:
- US-A1- 2008 078 805
- US-A1- 2010 237 132

## Beschreibung

Die vorliegende Erfindung betrifft ein vorzugsweise elektrochirurgisches Instrument, insbesondere für laparaskopische Eingriffe, gemäß dem Oberbegriff des Anspruchs 1.

### Hintergrund der Erfindung

Nach chirurgischer Entfernung eines Hohlgefäßabschnitts, z.B. bei einer Darmresektion aufgrund eines mit einem Tumor befallenen Darmteils, müssen die zwei Hohlgefäßschnitte an ihren eröffneten Enden wieder so miteinander verbunden werden, dass ein kontinuierlicher Verlauf entsteht. Man spricht hierbei von einer End-zu-End-Anastomose. Standardmäßig werden hierbei die beiden eröffneten Enden z.B. mit Klammernahtgeräten wieder aneinander genäht.

Insbesondere bei Dünn- und Dickdarmeingriffen kommt es zuweilen zu undichten Nahtverbindungen (Nahtinsuffizienz), welche mit einem schweren Krankheitsverlauf und auch einer hohen Sterberate verbunden sind.

Herkömmlich werden Wunden, beispielsweise bei Operationen, alternativ zum Klammern durch Vernähen mit Fäden verschlossen. Beide Techniken sind relativ aufwändig und belassen zumindest zeitweilig Fremdstoffe im Körper, die unter Umständen allergische Reaktionen hervorrufen können. Zudem steigt die Gefahr der versehentlichen Einbringung von Fremdstoffen, im ungünstigsten Falle Krankheitserregern oder Allergenen. Daher wird versucht, derartige Verfahren zu ersetzen und/oder zumindest in möglichst geringem Umfang einzusetzen.

Zur Herstellung einer chirurgischen Anastomose, also einer operativ hergestellten Verbindung zwischen Blutgefäßen, Nerven oder Hohlorganen wie vorstehend erörtert wurde als eine neuartige Technik das Versiegeln von Gewebe mit bipolarem, hochfrequentem Strom, die sogenannte "Tissue Fusion Technology" (TFT) entwickelt. Diese Technik kommt ohne im Körper verbleibende Fremdstoffe aus und sterilisiert zudem während des Versiegelungsvorgangs das Gewebe, so dass die Infektionsgefahr weiter reduziert werden kann.

Die Gewebefusion mittels Hochfrequenztechnik (HF) beruht auf der Denaturierung von Proteinen, die in vielen Geweben enthalten sind. Hierdurch ist es möglich, kollagenhaltige Gewebe zu verschweißen. Das Gewebe wird während des Schweißvorganges auf Temperaturen oberhalb der Eiweißdenaturierungstemperatur erhitzt und zusammen mit der intra- und extrazellulären Matrix in einen gelartigen Zustand gebracht. Nach Zusammendrücken der Gewebeflächen kühlt das verflüssigte Gewebe zu einer fusionierten Masse ab, was eine sichere Verbindung der Gewebe bewirkt.

Zum Verschweißen der Hohlgefäßschnitte wird das zwischen zwei Klemmbacken gefasste Gewebe mit einem bipolaren, hochfrequenten Strom beaufschlagt, der zwischen Elektroden an den beiden Klemmbacken fließt. Eventuell überschüssiges Gewebe, das in das Hohlorgan hineinragt, wird wie bei der Klammertechnik anschließend abgeschnitten, um einen störungsfreien Durchgang des Hohlorgans zu erhalten.

Damit es nicht zum Versagen der Versiegelung bzw. Verschweißung kommt, müssen die auf das Gewebe einwirkenden Parameter erfasst und geregelt werden. Um dies zu gewährleisten, benötigt man eine genaue Kontrolle von Temperatur, Gewebe-Klemmdruck, Gewebeimpedanz, Abstand und Lage der sich gegenüberliegenden Elektroden, etc.

Es ist wünschenswert, das zwischen den Klemmbacken gehaltene/eingeklemmte Gewebe überall gleichmäßig zu behandeln, so dass alle Bereiche zuverlässig erreicht werden und kein Bereich mit einem zu hohen Strom oder zu hohem Druck beaufschlagt wird. Dazu wird sichergestellt, dass die HF-Elektroden gleichmäßig voneinander beanstandet bzw. parallel zueinander ausgerichtet sind.

Die vorliegende Erfindung befasst sich vorwiegend mit der Kontrolle und Einstellbarkeit der Flächenpressung (Gewebe-Einklemmdruck/-kraft), die vor allem vor und während der Versiegelung sowie während des abschließenden Schneidens auf das Gewebe wirkt. Vorgestellt werden Lösungen für ein zirkuläres, ein lineares und ein laparoskopisches Versiegelungsinstrument. Darüber hinaus werden Möglichkeiten aufgezeigt, die Beherrschbarkeit des Verfahrens durch zusätzliche Sicherungseinrichtungen zu verbessern.

### Stand der Technik

Im Stand der Technik sind Klammernahtinstrumente bekannt, bei denen die Höhe der umgeformten Klammern einstellbar ist. Allerdings ist diese Technologie nicht direkt mit einer Einstellung der Flächenpressung vergleichbar, da auf diese Weise lediglich indirekt anhand des Unterschieds zwischen der Dicke des Gewebes und der Höhe der Klammern ein Zusammenpressen des Gewebes erfolgt. Der durch die gesetzten Klammern ausgeübte Druck ist somit nicht einstellbar und kann lediglich auf Grund von Erfahrungswerten bezüglich des Verhaltens des jeweiligen Gewebes vorab abgeschätzt werden. Während der Operation erhält der Chirurg darüber hinaus keine Rückmeldung über eine eventuelle mechanische Schädigung des Gewebes über die beim Setzen der Klammern zwangsläufig erfolgende hinaus.

Es sind folgende Patentanmeldungen bekannt, die einstellbare Abstände zum Halten von zu klammernden Geweben für zirkuläre Klammernahtinstrumente beschreiben:
Die EP2083710A1 offenbart eine Kassettenanordnung zum Tragen einer Klammerkassette. Die Kassettenanordnung kann in eine Schussposition zum Klammernsetzen gebracht werden. Ein Amboss, auf dem die Klammern gesetzt werden, wird funktionell mit einem Ambossverschluss gekoppelt. Der Ambossverschluss kann selektiv den Amboss in eine proximale Richtung hin zu der Kassettenanordnung bewegen, um einen Teil eines zu klammernden Gewebes durch die Kassettenanordnung und den Amboss einzuklemmen. Die Vorrichtung kann ferner ein Stellglied umfassen, das mit dem Ambossverschluss so zusammenwirkt, dass der Amboss in der proximalen Richtung bewegt wird, um die Klemmung zu erhöhen, wodurch das Gewebe zwischen der Kassettenanordnung und dem Amboss stärker zusammengepresst wird. Ein Element zum Erzeugen einer variablen Last kann den Druck auf das Gewebe zwischen dem Ambossverschluss und dem Stellglied vorwählen, bevor die Klammer gesetzt wird.
Die EP2055246A1 offenbart eine chirurgische Klammervorrichtung für kreisförmige Anastomosen. Die Vorrichtung umfasst eine Anzeige, die dem Chirurgen die Annäherung von Amboss und Kassettenanordnung und die Bereitschaft zum Klammernsetzen zeigt. Der Amboss ist nach dem Setzen abkippbar, um die Vorrichtung nach dem Klammern leichter aus dem Hohlorgan entfernen zu können.
Auch die EP2160984A2 offenbart eine chirurgische Klammervorrichtung, die im Hinblick auf die vorliegende Erfindung vergleichbar mit der Vorrichtung aus der EP2055246A1 ist.

Druckbegrenzungen werden in Instrumenten für minimalinvasive Chirurgie (MIC-Instrumenten) eingesetzt, um Gewebeschäden zu vermeiden.

Bei Koagulationsinstrumenten wie hier beschrieben kann ein gewünschter Abstand zwischen den Elektroden durch an den Klemmbacken angebrachte Abstandshalter eingehalten werden. Wenn jedoch auf den Klemmbacken eine größere Anzahl von Abstandshaltern vorgesehen ist, wie dies z.B. in EP 1 656 901 B1, EP1 952 777 A1, EP 1 372 507 A1 oder US 2004/122423 A1 vorgeschlagen wird, perforieren die Abstandshalter zwangsläufig das zu behandelnde Gewebe, da das Gewebe unter den Abstandhaltern bei geschlossenen Klemmbacken derart komprimiert wird, dass es zu dauerhaften Gewebeschädigungen kommt. Dies hat negative Auswirkungen auf das Ergebnis der Versiegelung.

Da derartige Abstandshalter bevorzugt aus elektrisch nicht leitendem Material sind, um einen Kurzschluss zwischen den HF-Elektroden zu vermeiden, entsteht im Bereich dieser Abstandshalter zudem ein sogenannter Koagulationsschatten, d.h. dass die Gewebeabschnitte im Bereich der oder unter den Abstandshaltern abgekapselt sind, somit nicht oder nur ungenügend mit Strom beaufschlagt werden und es dort zu keiner zufriedenstellenden Verschweißung der Gefäßabschnitte kommt. Außerdem hat es sich gezeigt, dass derartige elektrisch nichtleitende Abstandshalter insbesondere dann, wenn sie auf der Elektrode beispielsweise durch Kleben befestigt werden, leicht abplatzen können, um dann gegebenenfalls unbemerkt in den Patientenkörper zu gelangen. Darüber hinaus ist dann der vordefinierte Elektrodenabstand nicht mehr gewährleistet.

Moderne Klammernahtinstrumente am Markt weisen also eine einstellbare Klammerhöhe und eine Anzeige auf, ob die "richtige" Klammerhöhe eingestellt wurde. Allerdings wird dem Anwender keine Information darüber gegeben, wie sehr er das Gewebe beim Setzen oder Klammern komprimiert oder anderweitig beansprucht. Damit hat der Anwender auch keine Rückmeldung über eine eventuelle mechanische Schädigung des Gewebes. Darüber hinaus muss die Flächenpressung während des Verklammerns nicht in direkter Beziehung zu der Kraft stehen, die die gesetzte Klammer ausübt, weil sich das Gewebe insbesondere bei Komplikationen nach der Verbindung um die Klammer herum verändern kann. Beispielsweise kann es anschwellen oder sich entzünden. Bei Koagulationsinstrumenten wird die Einstellung des Abstands zwischen den Klemmbacken, die funktionell dem Amboss und dem Gegenhalter (meist der Klammerkassette) der klammernden Instrumente entsprechen, über Abstandshalter gelöst.

Die US 2010/0237132 A1 wird als nächstkommender Stand der Technik angesehen, der die Grundlage für den Oberbegriff des Anspruchs 1 bildet.

Die EP2079372 A1 offenbart ein Gerät mit einer Kassetten-Baugruppe zur Aufnahme eines Klammermagazins. Ein Amboss ist mit einem Ambossverschluss gekoppelt. Der Ambossverschluss kann selektiv den Amboss in eine proximale Richtung hin zu der Kassetten-Baugruppe bewegen, um einen Teil eines zu klammernden Gewebes zwischen der Kassetten-Baugruppe und dem Amboss einzuklemmen. Die Vorrichtung kann ferner eine Kompression des Gewebes zwischen dem Ambossverschluss und dem Stellglied begrenzen, um eine weitere Bewegung des Amboss in der proximalen Richtung hin zu der Kassetten-Baugruppe zu verhindern, wenn die vorbestimmte Höhe der Kompression erreicht ist.

Für zirkuläre Klammernahtinstrumente sind außerdem folgende Druckschriften bekannt, die Sicherheitsvorkehrungen betreffen:
Die US2009230170A1 offenbart eine chirurgische Klammervorrichtung mit einem Gehäuse, einem länglichen Abschnitt, einem distalen Endteil, und einen beweglichen Griff, der mit dem Gehäuse mechanisch zusammenwirkt und zwischen einer ersten offenen Position und einer zweiten Position bewegbar ist, die zum Einklemmen eines Gewebes gegen das distale Endteil führt. Ein Ratschenmechanismus wirkt mechanisch mit dem beweglichen Handgriff zusammen, um verhindern, dass der bewegliche Handgriff sich in Richtung der ersten offenen Position bewegt, bevor der bewegliche Handgriff eine vorbestimmte Position erreicht hat.
Die EP2233084A1 zeigt eine chirurgische Klammervorrichtung mit einem Ambossverriegelungssystem. Dadurch wird eine unerwünschte Verschiebung des Amboss in axialer Richtung verhindert.

Es sind jedoch keine vergleichbaren Sicherheitsvorkehrungen beim Einsatz von HF-Versiegelungsinstrumenten bekannt. Darüber hinaus sind die Anforderungen an die Sicherheitstechnik auf Grund der völlig unterschiedlichen Gewebeverbindungsarten nicht einfach übertragbar.

### Kurzbeschreibung der vorliegenden Erfindung

Somit ist es die Aufgabe der Erfindung, ein Versiegelungsinstrument zu schaffen, das Gewebeschäden bei der Anastomose minimieren kann.

Diese Aufgabe wird durch ein chirurgisches Instrument nach Anspruch 1 gelöst. Vorteilhafte Weiterbildungen und ggf. unabhängig zu beanspruchende weitere Aspekte der Erfindung sind Gegenstand der Unteransprüche.

Erfindungsgemäß wurde erkannt, dass Gewebeversiegelungen im Unterschied zum Setzen von Klammern wie in den vorstehend erläuterten Druckschriften erörtert eine über eine ausreichend lange Zeit aufrecht erhaltene, definierte Flächenpressung bzw. einen definierten Flächenpressungsbereich brauchen. Daher ist es notwendig, eine entsprechende Klemmdruck-Einstelleinrichtung an den Instrumenten vorzusehen. Auf diese Weise können die vorstehend erörterten Probleme, die mit Abstandshaltern auftreten, vermieden oder zumindest reduziert werden, weil keine Abstandshalter eingesetzt werden müssen, die das Gewebe partiell zusammendrücken/perforieren können, und selbst beim Einsatz von Abstandshaltern nur eine vorab definierte Kraft auf das Gewebe wirkt, die nicht zu Schädigungen führt.

Die hier beschriebene Lösung zeigt das erste bekannte Lösungskonzept für ein vorzugsweise zirkuläres Versiegelungsinstrument. Zudem sind vergleichbare Mechaniken von Klammernahtinstrumenten nicht bekannt, da sie, wie bereits erörtert, dort nicht in dieser Art gebraucht werden. Aufgrund der Tatsache, dass insbesondere lineare und laparoskopische Versiegelungsinstrumente für eine Vielzahl von Gewebearten und Indikationen verwendet werden, schafft die Erfindung eine Einstellmöglichkeit für den Anwender, die eine ideale Flächenpressung für jede Anwendung ermöglicht. Bisher bekannte Instrumentenkonzepte sehen die oben gezeigten Einstellmöglichkeiten nicht vor, was die Einsatzmöglichkeit dieser Instrumente auf bestimmte Gewebearten und Indikationen beschränkt.

Ausgehend vom vorstehend genannten Stand der Technik wird erfindungsgemäß durch den Einbau einer Druckeinstelleinheit in ein chirurgisches Instrument vorzugsweise zur Versiegelung mit bipolarer Hochfrequenztechnologie eine reproduzierbare Gewebeversiegelung erreicht. Es wird gewährleistet, dass das Gewebe mit einer definierten Anpresskraft zwischen den beiden Elektroden gehalten wird. Da durch die elektrische Versiegelung eine gleichmäßigere Verteilung von Wundbelastungen erreicht wird, kann ein gleichbleibend guter Gewebeverbund geschaffen und die Heilung beschleunigt werden.

Erfindungsgemäß kann der Anwender aktiv die am Gewebe wirkende Flächenpressung in einem vordefinierten Bereich einstellen. In einer bevorzugten Ausführungsform kann der Anwender durch eine Anzeigemechanik direkt kontrollieren, ob die richtige Flächenpressung eingestellt ist. Zu starke Quetschung des Gewebes wird durch die definierte Krafteinwirkung vermieden. Allgemein wird durch die Einstellbarkeit der Flächenpressung der Einsatzbereich der Instrumente ausgeweitet. Die Anwender- und Patientensicherheit wird damit erhöht.

Eine zusätzliche Anzeige am proximalen Ende insbesondere bei einem zirkulären Instrument nach einer weiteren Ausführungsform ist eine weitere Hilfe für den Anwender, da der Bereich der Anzeigeskala oft nicht gut eingesehen werden kann. Die Flächenpressung wird durch einen einstellbaren Federmechanismus aufgebracht. Federn wie Gasdruck-, Schraubenzug-, Schraubendruck-, Teller- oder Blattfedern sind bekannte mechanische/pneumatische Elemente, um Kräfte auszuüben. Diese Federn werden vorgespannt, was beispielsweise über eine Vorspannschraube erfolgen kann. Auf diese Weise kann über die vorgewählte Federvorspannung, die dadurch erzeugte Federkraft bei einem gegebenen Abstand und die Fläche der Klemmbacken die Flächenpressung auf das Gewebe einfach und sicher reproduzierbar eingestellt werden.

Alternativ kann die Kraft zum Erzeugen der Flächenpressung auch auf andere Arten aufgebracht werden. Beispielsweise ist es bei telechirurgischen Instrumenten, die erfindungsgemäß ausgestattet sind, vorstellbar, die Flächenpressung über eine motorische Ansteuerung einzustellen. Auch in diesem Fall kann eine Feder vorgesehen sein, die entweder eine Gegenkraft zu einer Motorkraft aufbaut, die das Gewebe klemmt, oder die selbst motorisch vorgespannt wird.

Es ist auch denkbar, einen Excenter mit den Klemmbacken so zu koppeln, dass die Flächenpressung durch Drehen des Excenters einstellbar ist. Im Vergleich mit der vorstehend vorgeschlagenen Anordnung mit Feder und Vorspannschraube hat die Einstellung über einen Excenter den Vorteil, in der Regel platzsparender und mit geringerer Bewegungsamplitude nachstellbar zu sein.

Besonders vorteilhaft kann die gesamte Einstellmechanik in einem Teil untergebracht werden. Auf diese Art ergeben sich Vorteile hinsichtlich des kompakten Aufbaus und einfacheren Ersatzes bei eventuellen Defekten.

Ferner kann das erfindungsgemäße Instrument noch derart weitergebildet werden, dass Sicherheitsmechanismen vorgesehen werden, um die Versiegelung zu gewährleisten, ohne das Gewebe (beispielsweise durch eine falsch eingestellte Flächenpressung oder ein nicht korrekt eingeführtes Instrument) versehentlich zu schädigen.

Konkreter wird die gestellte Aufgabe gelöst durch ein chirurgisches Instrument vorzugsweise der HF-Bauart bzw. der zirkulären HF-Bauart mit zwei Gewebe- oder Klemmbranchen (auch Instrumentenkopf und Amboss), die mit Elektroden besetzt sind, von denen zumindest eine relativ zur anderen bewegbar ist und die über einen Betätigungsmechanismus (unter dazwischen Einklemmen von Körpergewebe) mit einem vorbestimmten oder vorbestimmbaren Anpressdruck an die andere Gewebebranche anlegbar ist. Erfindungsgemäß ist eine Klemmdruck-Regeleinrichtung bzw. Klemmdruck-Einstelleinrichtung (oder auch Klemmdruck-Bereitstellungseinrichtung) vorgesehen, die in einem Kraft- oder Momentübertragungszug zwischen dem Betätigungsmechanismus und der zumindest einen bewegbaren Gewebebranche (seriell) zwischengeschaltet ist.

Die Klemmdruck-Elnstelleinrichtung ist dafür angepasst, bei einem vorbestimmten oder vorbestimmbaren Betätigungsbetrag des Betätigungsmechanismus eine vorbestimmte oder vorbestimmbare Kraft (Moment) in den Kraft- oder Momentübertragungszug einzuleiten, entsprechend welcher ein zugehöriger Klemmdruck (Klemmkraft) auf das eingeklemmte Gewebe ausgeübt und entsprechend der Betätigungsdauer auch gehalten wird. Die Klemmdruckregel-/Einstelleinrichtung hat einen (hubartig bzw. linear verschiebbar wirkenden) Kraftspeicher, der durch den Betätigungsmechanismus um einem definierten/definierbaren (Verschiebe-)Betrag oder innerhalb eines definierten/definierbaren Betragsbereichs aufladbar ist, bei welchem eine/ein daraus resultierende(s) sowie über den Kraft- oder Momentübertragungszug übertragene(s) Betätigungskraft oder Betätigungsmoment den vorbestimmten oder vorbestimmbaren Anpressdruck erzeugt. In anderen Worten ausgedrückt ist es vorgesehen, die Regel-/Einstelleinrichtung mit einem Energiespeicher (Druckfeder) auszubilden, der bei einem bestimmten Verformungsweg eine zugehörige Kraft auf ein nachgeschaltetes Kraftübertragungssystem ausübt, derart, dass dann, wenn der Energiespeicher (Feder) mittels des Betätigungsmechanismus über ein bestimmtes Maß (Federweg) von außen visuell erkennbar deformiert wird, die daraus resultierende Deformationskraft über das Kraftübertragungssystem auf die Instrumentenbranchen als Klemmkraft angelegt wird. Die Klemmdruckregel-/Einstelleinrichtung wirkt somit nach dem Prinzip eines elastisch nachgebenden Hubzylinders, dessen elastische Nachgiebigkeit in Axialrichtung einstellbar ist. Somit wird die Klemmkraft nicht direkt über das Betätigungselement sondern indirekt über den Energiespeicher in Abhängigkeit seines Deformationswegs sowie seiner Deformationseigenschaft erzeugt und ist damit reproduzierbar. Die vorliegende Erfindung unterscheidet sich damit auch nachdrücklich von einem einfachen Auslösemechanismus (Überlastschutz), der beispielsweise zwischen Betätigungselement und Kraftübertragungssystem zwischengeschaltet ist und bei Erreichen einer eingestellten maximalen Betätigungskraft den Kraftfluss unterbricht. Ein solcher Auslösemechanismus würde zwar eine Überbelastung des Gewebes vermeiden, er könnte jedoch nicht gewährleisten, dass eine bestimmte Klemmkraft auch tatsächlich erreicht und über einen bestimmten Zeitraum gehalten wird.

An dem Kraftspeicher kann zusätzlich eine Sicherheitseinrichtung in Form eines Ladebetragssensors angeordnet sein, der ein Aktivieren des chirurgischen Instruments zum Auslösen einer bestimmten Instrumentenfunktion (z.B. HF-Stromzufuhr) erst dann zulässt, wenn ein bestimmter Ladebetrag erreicht ist. Weiter vorzugsweise ist der Kraftspeicher eine (Druck-)Feder oder Federanordnung, die betragsentsprechend deformierbar ist. Hierbei hat es sich als vorteilhaft erwiesen, wenn der Kraft- oder Drehmomentübertragungszug zumindest zwei gegeneinander bewegbare Elemente beispielsweise zwei teleskopartig (axial) relativ bewegbare Schubelemente hat, zwischen denen die Feder zwischengefügt ist. Wird somit das eine (z.B. äußere) Schubelement axial bewegt, wird das andere (z.B. innere) Schubelement ohne Relativversatz über die Zwischenfederanordung verschoben, solange kein Bewegungswiderstand auf das andere (innere) Schubelement angelegt wird. Sobald jedoch das andere (innere) Schubelement auf einen Widerstand trifft und ggf. gestoppt wird, findet ein Axialversatz zwischen beiden Schubelementen statt, wodurch die Zwischenfederanordung gespannt wird und eine Axialkraft auf das andere (innere) Schubelement ausübt. Die axial wirkende Federanordnung hat sich dabei als besonders vorteilhaft (beispielsweise gegenüber einer Drehfeder) erwiesen, da sie über einen vergleichsweise langen Federweg eine im Wesentlichen lineare Federkennlinie zeigt. Hierbei können die gegeneinander bewegbaren Elemente jeweils mit Federsitzen ausgebildet sein, von denen zumindest einer vorzugsweise verstellbar ist, um im Rahmen eines maximal vorgesehenen (immer gleichbleibenden) Betätigungswegs oder Betätigungsbetrags des Betätigungsmechanismus unterschiedliche Deformationsbeträge (und damit unterschiedliche Druckkräfte) der Feder zu bewirken.

Des Weiteren kann der Kraftspeicher mit einer Anzeigeeinrichtung mechanisch gekoppelt sein, über die der Ladebetrag bzw. über den Betätigungsmechanismus bewirkter Deformationsbetrag detektier- und vorzugsweise visuell als aktueller Branchen-Anpressdruck anzeigbar ist. Diese Anzeigeeinrichtung kann ein mit einem der Federsitze (Betätigungsmechanismus-zugewandter Federsitz) wirkgekoppelter Mitnahmeriegel oder Zeiger sein, der mit Beginn einer vom Betätigungsmechanismus ausgelösten Federdeformation von dem Federsitz mitgenommen wird und dadurch diesen Deformationsweg abgreift und vorzugsweise anzeigt. Auch kann zusätzlich oder alternativ ein Kraft- oder Drehmoment-Begrenzungsmittel vorzugsweise in Form einer Rutschkupplung vorgesehen sein, das zwischen dem Betätigungsmechanismus und dem Kraftspeicher angeordnet ist. Das Kraft- oder Drehmoment-Begrenzungsmittel kann optional einstellbar sein, wobei die Einstellung wiederum anzeigbar ist, um daraus den erreichbaren Klemmdruck zu erhalten.

Abschließend sei darauf hingewiesen dass eine Feder nur eine Form der konstruktiven Ausgestaltung des Kraftspeichers darstellt. Statt dessen können als Kraftspeicher auch andere Lösungen wie beispielsweise ein Elektromagnet, eine Spule etc. vorgesehen sein, deren Magnetfeder über den Betätigungsmechanismus einstellbar sind, um eine entsprechende Kraft/Drehmoment in den Übertragungszug einzuspeisen.

Die Erfindung wird nachstehend anhand von mehreren Ausführungsbeispielen unter Bezugnahme auf die begleitenden Figuren genauer erläutert. In den Figuren zeigt:
Figur 1 eine Längsschnittansicht einer beispielhaften Klemmdruck-Regeleinrichtung (Drucksteuermechanik) am proximalen Ende eines zirkulären elektrochirurgischen Instruments gemäß einem ersten Ausführungsbeispiel;
Figur 2 eine Längsschnittansicht eines Griffstücks des Instruments gemäß der Fig. 1 mit einer darin eingebauten beispielhaften (visuellen) Klemmdruck-Anzeige, welche eine (mechanische) Wirkverbindung zur Drucksteuermechanik hat;
Figur 3 eine beispielhafte Anzeigeskala des Anzeige gemäß der Fig. 2;
Fig. 4 einen Gesamtlängsschnitt des Griffstücks gemäß der Fig. 3 einschließlich der Manipulatoren zur Betätigung der Instrumentenkopf-Funktionen (Effektoren),
Fig. 5 den Schaft-/Instrumentenkopf des elektrochirurgischen Instruments gemäß dem ersten Ausführungsbeispiel mit seinen Funktionselementen
Fig. 6 eine Prinzipdarstellung des elektrochirurgischen Instruments gemäß dem ersten Ausführungsbeispiels,
Fig. 7 eine Draufsicht des Instrumentengriffs gemäß der Fig. 4,
Fig. 8 die Seitenansicht eines elektrochirurgischen Instruments gemäß einem bevorzugten Ausführungsbeispiels der vorliegenden Erfindung,
Fig. 9 einen Seitenriss eines Handstücks des zweiten Ausführungsbeispiels im Bereich der Klemmdruck-Regeleinrichtung,
Fig. 10 eine Längsschnittansicht eines elektrochirurgischen Instruments der linearen Bauart gemäß einem weiteren bevorzugten Ausführungsbeispiel der Erfindung,
Fig. 11 die Prinzipdarstellung der Einstelleinrichtung einer Klemmdruck-Regeleinrichtung,
Fig. 12a-12c Varianten der Anordnung und konstruktiven Ausführung einer erfindungsgemäßen Klemmdruck-Regeleinrichtung am ersten Ausführungsbeispiel,
Fig. 13 eine Längsdarstellung eines weiteren elektrochirurgischen Instruments der linearen Bauart gemäß einem vierten Ausführungsbeispiel ,
Fig. 14 eine Längsdarstellung eines Spannmechanismus des elektrochirurgischen Instruments gemäß Fig. 13 in Vergrößerung,
Fig. 15 eine Längsdarstellung eines zu Fig. 14 alternativen Spannmechanismus des elektrochirurgischen Instruments gemäß Fig. 13 und
Fig. 16 eine Rückansicht des Spannmechanismus gemäß Fig. 15.

Angelehnt an das Design und die Funktion insbesondere von zirkulären Klammernahtinstrumenten wurde vorliegend ein Versiegelungsinstrumenten-Prinzip entwickelt, das dem Anwender die grundsätzliche Möglichkeit bietet, eine definierte Flächenpressung (Klemmdruck) an den Instrumentenklemmbacken/Branchen einzustellen. Der für das Behandlungsergebnis optimale, einstellbare Bereich von vorzugsweise 0,7 N/mm² bis 2,0 N/mm² wurde durch Vorversuche definiert und soll durch die erfindungsgemäße Klemmdruck-Regeleinrichtung sicher einstellbar sein. Dabei sei aber darauf hingewiesen, dass auch andere Werte für die bevorzugten Klemmdrücke vorstellbar sind in Abhängigkeit des zu behandelnden Körpergewebes.

Der erfindungsgemäßen Klemmdruck-Regeleinrichtung liegt nunmehr der allgemeine Gedanke zugrunde, dass eine Vorspannfeder als eine einfache Variante eines Kraftspeichers mit vorbekannter Federcharakteristik bei einem bestimmten Verformungsweg eine zugehörige konkrete Federkraft ausübt, die aus der Federcharakteristik berechenbar und immer wieder reproduzierbar ist. Wird also eine solche Vorspannfeder über einen bestimmten Federweg deformiert, kann aus dem visuell leicht ablesbaren (darstellbaren) elastisch deformierten Federweg ein Rückschluss auf die dadurch erzeugbare/erzeugte Federkraft gezogen werden, die dann beispielsweise an die Instrumentenklemmbacken/Branchen zur Komprimierung von darin eingespanntem Körpergewebe anlegbar ist. Dies wiederum ermöglicht es prinzipiell unter Berücksichtigung des Wirkungsgrads des Kraft-/Momentübertragungszugs von der Feder auf die Klemmbacken/Branchen, einer bestimmten Federvorspannkraft (entsprechend dem visuell anzeigbaren Feder-Deformationsweg) eine bestimmte Flächenpressung zwischen den Klemmbacken/Branchen zuzuordnen. Das bedeutet, dass der aktuelle Druck indirekt über den elastischen Feder-Deformationsweg präzise darstellbar ist. Dabei stellt die Feder natürlich nur eine Variante für einen Kraftspeicher dar. Es ist vorstellbar, eine elektrische Energiequelle anstelle der Mechanischen vorzusehen und die aktuell abgegebene Energie in Abhängigkeit der Betätigungsbetrags einer Betätigungsvorrichtung zu regeln. Diese abgegebene Energie könnte in eine Betätigungskraft vergleichbar zur Federvorspannkraft gewandelt werden. Im Nachfolgenden wird aber ausschließlich auf eine Feder als Kraftspeicher Bezug genommen.

Das vorstehende Prinzip ist im übrigen bei allen Arten von chirurgischen, Gewebe klemmenden Instrumenten anwendbar, unabhängig davon, ob die Kraft zur Deformation der Vorspannfeder manuell oder motorisch aufbringbar ist. Darüber hinaus lässt sich dieses Funktionsprinzip sicherungstechnisch beliebig erweitern, indem beispielsweise zwischen der Vorspannfeder und dem Mechanismus zur Vorspannung der Feder ein Kraft-/Drehmomentbegrenzer beispielsweise in Form einer Rutschkupplung zwischengefügt ist oder indem der motorische Antrieb das Überschreiten einer maximalen Antriebskraft selbständig unterbindet beispielsweise wie ein Schrittmotor oder ein Hydraulik-/Pneumatikantrieb mit Druckbegrenzungsventil.

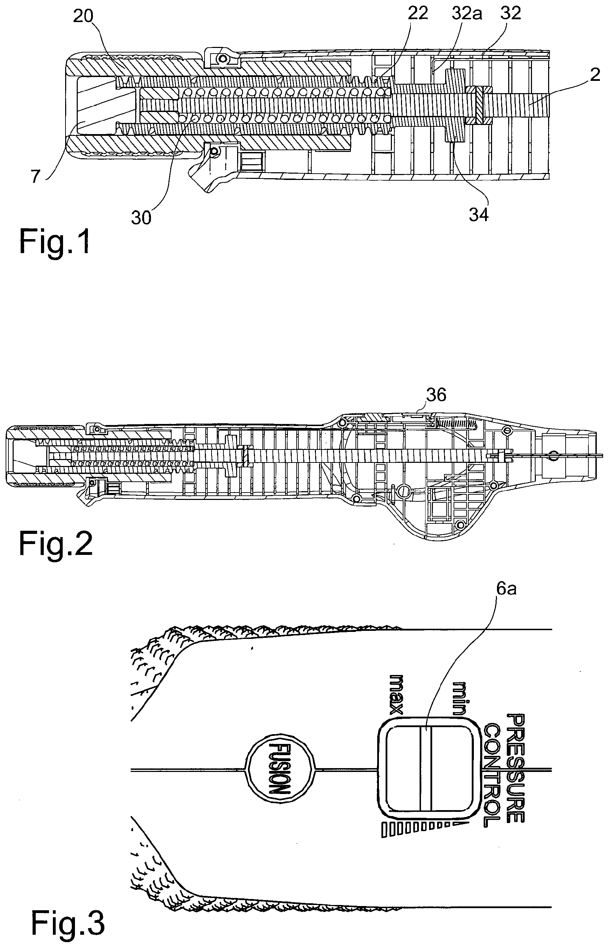

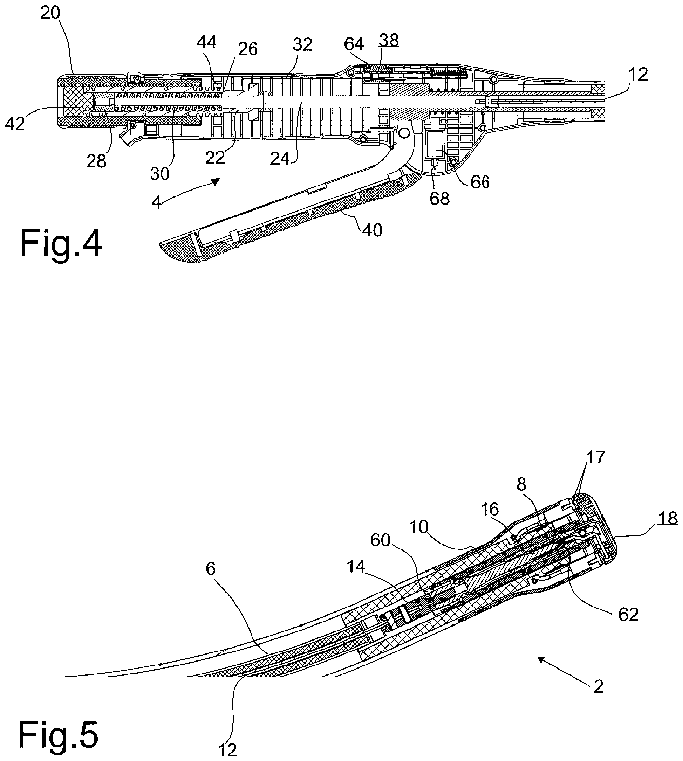

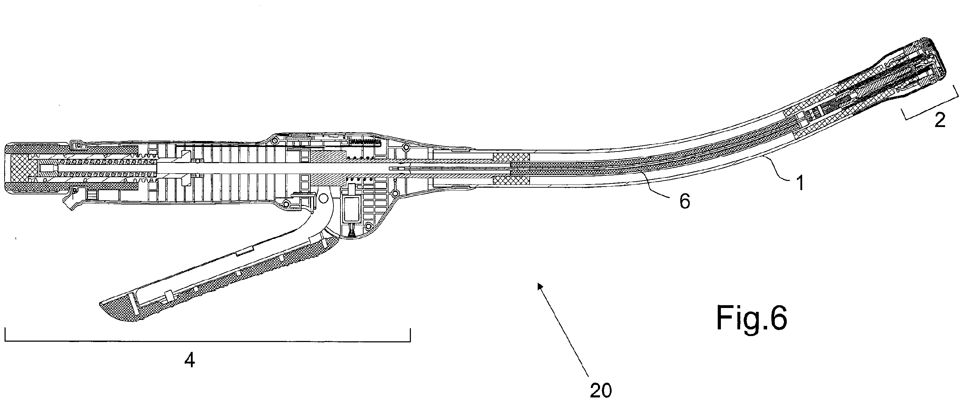

Wie aus Fig. 1 in Kombination mit den Fig. 5 und 6 erkennbar ist, betrifft ein erstes Ausführungsbeispiel im Konkreten ein zirkulares elektrochirurgisches (HF-) Instrument mit einem vorzugsweise gebogenen Instrumentenschaft 1, an dessen distalem (körperzugewandten) Ende ein Schaftkopf 2 und an dessen proximalem (körperabgewandten) Ende ein Handgriff oder Griffstück 4 angeordnet ist. Innerhalb des Schafts 1 ist ein Zug-/Druck-Profilelement 6 axialbewegbar geführt, das an seinem distalen Ende mit einer axial wirkenden Kreis-Schneidklinge 8 gekoppelt ist, welche im Schaftkopf 2 über eine Klingenhalterung 10 axialverschiebbar gelagert ist. Innerhalb des Zug-/Druck-Profilelements 6 ist ferner eine Zug-/Druckwelle/Stange, d.h. eine Zug-/Druck-Einheit vorzugsweise in der Ausführung eines oder mehrerer Blechbänder 12 relativ bewegbar gelagert, die an ihrem distalen Ende mit einem sogenannten Trokardorn 14 fest gekoppelt ist, der innerhalb der Klingenhalterung 10 relativ verschiebbar gelagert und endseitig in einem axialen Hohlschaft 16 eines distalen Amboss (Elektrodenplatte) 18 eingeführt ist. Der Amboss 18 bildet eine Art Teller oder Schirm und liegt mit seiner einen Flachseite der Schaftkopfstirnseite axial gegenüber und kann über die Zug-/Druck-Stange 12 und den Hohlschaft 16 relativ zum Schaftkopf 2 axial bewegt werden. Die Stirnseite des Schaftkopfs 2 wie auch die zugewandte Flachseite des Amboss 18 sind mit Elektroden 17 einer bipolaren HF-Strom-Beaufschlagungseinrichtung besetzt, welche an elektrische Leitungen angeschlossen sind, die innerhalb des Instrumentenschafts 1 vorzugsweise des Zug-/Druck-Profilelements 6 verlegt sind. Darüber hinaus dienen der Amboss 18 und der Schaftkopf 2 dazu, Körpergewebe kreisförmig dazwischen axial einzuklemmen und mit einem vorbestimmten Druck zusammenzupressen.

Zur Betätigung/Axialverschiebung des Amboss 18 bezüglich des Schaftkopfs 2 befindet sich am proximalen Ende des als erstes Ausführungsbeispiel dargestellten (zirkularen) Instruments bzw. von dessen Griffstück 4 eine längs der Griffstücklängsachse drehbar gelagerte Drehmutter/Handrad/Drehknopf 20 mit einem Innengewinde, das mit einer axial bewegbar jedoch drehfest gelagerten Hohlwelle/Spindel 22 mit Außengewinde in Kämmeingriff ist, in der wiederum eine Zug-/Druck-Stange 24 gelagert ist, die mit der Zug-/Druck-Einheit 12 verbunden ist. Durch Drehen der Drehmutter 20 wird demzufolge eine geführte Axialbewegung der Hohlwelle 22 ausgelöst.

Wie insbesondere aus der Fig. 1 entnehmbar ist, hat die Hohlwelle 22 einen inneren Federsitz in Form einer inneren Ringschulter 26, die am distalen Endbereich der Hohlwelle 22 angeordnet ist und in der die Zug-/Druck-Stange 12, 24 relativ gleitgeführt ist. Die Zug-/Druck-Stange 12, 24 hat an ihrem proximalen Ende ebenfalls einen Federsitz in Form einer äußeren Ringschulter 28, die ebenfalls auch als Führung/Gleitlager zur axialen Gleitführung der Zug-/Zug-Stange 12, 24 innerhalb der Hohlwelle 22 dient. Zwischen den beiden Federsitzen ist eine Druckfeder/Spiralfeder 30 eingesetzt, welche die Zug-/Druck-Stange 12, 24 umgibt und eine Axialbewegung der Hohlwelle 22 auf die Zug-/Druck-Stange 12, 24 überträgt.

Wie aus der Fig. 2 und 3 erkennbar ist, ist in der Griffschale des Handgriffs/Griffstücks 4 ein Anzeigeriegel 32 axialverschiebbar gelagert mit einer Eingriffskralle/-haken 32a, die mit einem Mitnahmevorsprung 34 an der Hohlwelle 22 dann in Eingriff kommt, wenn diese eine Axialposition im Griffstück (Griffschale) 4 erreicht hat, bei der der Amboss 18 gerade auf der Stirnseite des Schaftkopfs 2 zu liegen kommt oder unmittelbar vorher (in Abhängigkeit der zu erwartenden Gewebedicke des einzuspannenden Körpergewebes).

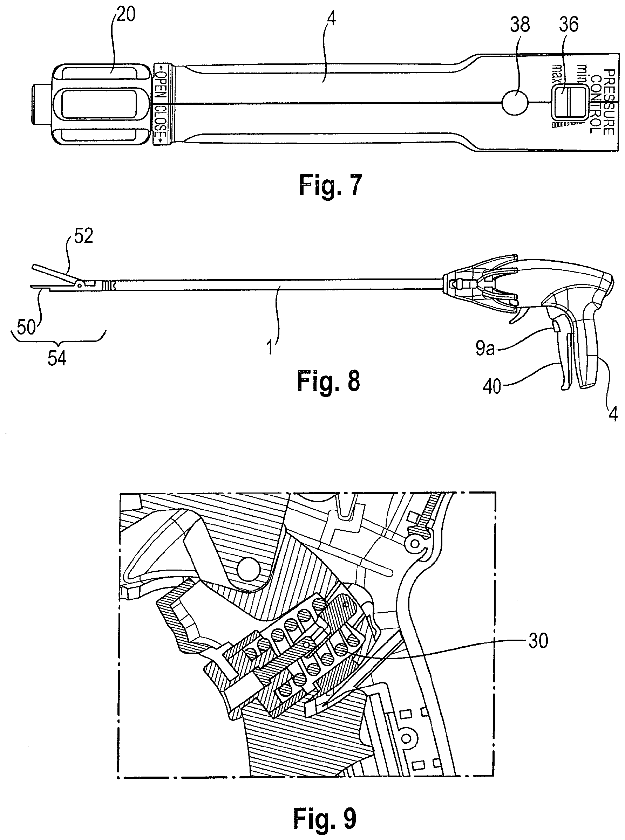

Bis zu dieser Axialposition der Hohlwelle 22 erfolgt die Axialbewegung des Amboss 18 über die Zug-/Druck-Stange 12 und die Druckfeder 30 noch im Wesentlichen kraftlos, ohne die Druckfeder 30 zu komprimieren. Ab dieser Axialposition bewegt sich der Amboss 18 jedoch nicht mehr oder unwesentlich in Abhängigkeit der Komprimierung des eingespannten Körpergewebes, sodass ein Weiterdrehen der Drehschraube 20 (Betätigungsmechanismus) und die dadurch ausgelöste Weiterverschiebung der Hohlwelle 22 im Wesentlichen ausschließlich durch eine Deformation der Druckfeder 30 kompensiert wird. Dieser Deformationsweg wird nunmehr durch ein Mitverschieben des Anzeigeriegels 32 detektiert und in einem Anzeigefenster 36 gemäß der Fig. 7 kenntlich gemacht.

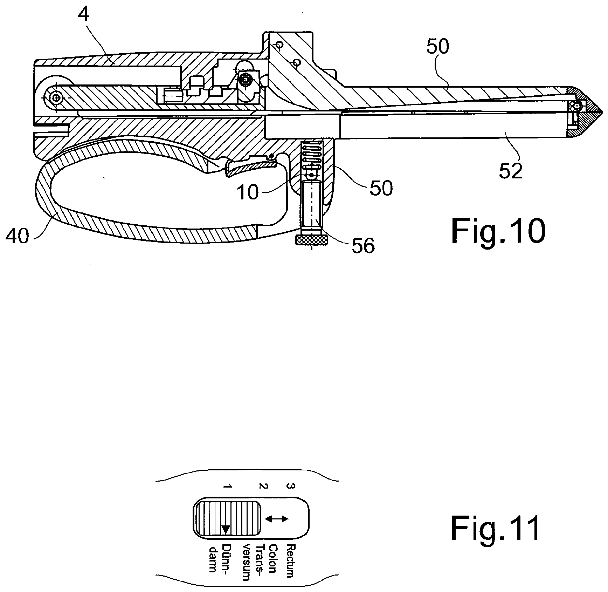

Dieses Anzeigefenster 36 hat vorzugsweise zwei Skalenwerte, nämlich "min" und "max", zwischen denen sich der Anzeigeriegel 32 befinden muss, um eine ausreichende Federdeformation vorauszusetzen. Da bei dieser Federdeformation die Feder-Vorspannkraft über den einen Federsitz 28 auf die Zug-/Druck-Stange 12 aufgebracht wird, wird diese über die Stange 12 auf den Amboss 18 übertragen, der dann mit einem entsprechenden Klemmdruck das Körpergewebe zwischen sich und dem Schaftkopf 2 definiert einspannt. Weiterhin kann das Anzeigefenster wie in Fig. 11 dargestellt ausgeführt sein. Hierbei würde der Riegel 32 anzeigen, dass die korrekte Flächenpressung für ein bestimmtes Gewebe eingestellt wurde.

Jetzt können die Elektroden 17 über einen HF-Auslösetaster 38 (siehe insbesondere Fig. 4) am Griffstück 4 sowie die daran angeschlossenen elektrischen Leitungen mit einem HF-Strom beaufschlagt werden. Sobald die Strombeaufschlagung beendet ist, wird ein weiterer, am Griffstück 4 gelagerter Scherengriff oder Griffbügel (Handhebel) 40 betätigt, der mit dem Zug-/Druck-Profilelement 6 gekoppelt ist, um dieses innerhalb des Instrumentenschafts 1 zu verschieben und dadurch die distal daran angeschossene Kreis-Schneidklinge 8 in Richtung hin zum Amboss 18 axial vorzuschieben. Diese Klinge 8 trennt dabei radial nach innen vorragendes, überschüssiges Körpergewebe ab.

Die Vorteile der erfindungsgemäßen Klemmdruck-Einstelleinrichtung gemäß der vorstehenden Beschreibung des ersten bevorzugten Ausführungsbeispiels der Erfindung lassen sich wie Folgt zusammenfassen:
Wenn der Amboss 18 mit den daran befindlichen Elektroden (-platten) eingezogen wird und am Instrumentenkopf 2 aufliegt (oder kurz davor), beginnt die Druckfeder 30 zwischen der Hohlwelle 22 und der Zug-/Druck-Stange 12, 24 zu wirken. Der Anwender kann nun über einen definierten Federweg die daraus resultierende Flächenpressung am Kopf steuern. Die direkte Rückmeldung über seine Einstellung erhält er durch den vorzugsweise als Anzeigeblech ausgebildeten Riegel 32, der von der Hohlwelle (Hohlspindel) 22 mitgenommen wird, wobei am Riegel 32 beispielsweise ein Zeiger optional angebracht sein kann. Der Riegel, bzw. das Anzeigeblech 32 ist durch das Anzeigefenster 36 im Handgriff 4 sichtbar, das insbesondere gemäß der Fig. 7 vorzugsweise an der Griffoberseite angeordnet ist und so im Gebrauch gut ablesbar ist. Eine zusätzliche Anzeigemöglichkeit kann über eine Endkappe 42 der Zug-/Druck-Stange 12, 24 realisiert werden, die sich bei beginnender Deformation der Druckfeder 30 (beginnender Relativbewegung zwischen Hohlwelle 22 und Zug-/Druck-Stange 12) allmählich am distalen Ende der Hohlwelle 22 aus dieser herausbewegt und somit bei der Einstellung des zulässigen Bereichs der Flächenpressung sichtbar wird.

Optional kann in der Griffschale ein Anschlag 44 vorgesehen sein, der mit der Hohlwelle (Hohlspindel) 22 bei einer bestimmten Axialposition in Anlage kommt. Diese Anschlag 44 verhindert in dieser Ausführungsform, dass die maximal zulässige Pressung überschritten wird.

Das Gewinde der Spindel 22 kann auch Bereiche unterschiedlicher Steigungen aufweisen. Durch unterschiedliche Steigungen oder entsprechende Getriebe können beispielsweise ein fein einstellbarer Bereich in der Mitte und ein schnell einstellbarer Bereich außerhalb der voraussichtlich "idealen" Pressung geschaffen werden. In anderen Worten ausgedrückt kann die Hohlwelle 22 (Hohlspindel) einen Bereich mit großer Steigung haben für ein schnelles Bewegen des Amboss 18, solange, bis dieser nahe dem Schaftkopf 2 ist und einen Bereich mit kleiner Steigung haben für ein langsames Deformieren der Druckfeder 30, wenn der Amboss 18 am Schaftkopf 2 anliegt (oder kurz davor).

Obwohl dies hier nicht im Einzelnen gezeigt wird, kann anstelle oder zusätzlich zu der Gewindespindel (Hohlwelle) auch das Wirkprinzip eines auslösenden oder eines anzeigenden Drehmomentschlüssels verwendet werden, um eine einstellbare Flächenpressung zu realisieren. Hierbei würde beispielsweise die Drehmutter über eine Rutschkupplung oder einen derartigen Auslösemechanismus mit der Hohlspindel gekoppelt sein, der beim Überschreiten der maximal zulässigen Flächenpressung durchrutschen und somit einen weiteren Druckaufbau verhindern würde. Ebenso ist - beispielsweise mit Hilfe von Dehnungsmessstreifen an einem Torsionsstab im Instrument eine (zusätzliche) elektronische Auswertung der Flächenpressung denkbar, die dann auch motorisch steuer- oder regelbar sein kann, insbesondere bei roboterunterstützten Operationstechniken wie in der Telechirurgie.

Bei einer in Fig. 8 und 9 gezeigten zweiten Ausführungsform, die an einem laparoskopischen Instrument mit scheren- oder zangenartigen Instrumentenbranchen am Schaftkopf dargestellt wird, befindet sich die Druckfeder 30 im beweglichen Hebel 40 des Handgriffs 4, der in diesem Fall zum Öffnen und Schließen der Zangenbranchen 50, 52 mit diesen über ein Schaft-Getriebezug gekoppelt ist. Das in Fig. 8 gezeigte Instrument ist im Gegensatz zur ersten Ausführungsform demnach kein zirkuläres Versiegelungsinstrument, sondern ein lineares Versiegelungsinstrument bei dem die Elektroden längs der Zangenbranchen 50, 52 angeordnet sind.

Über eine Einstellmöglichkeit an dem Hebel 40 kann der Anwender die wirkende Flächenpressung zwischen den Klemmbacken 50, 52 im distalen Maulteil 54 verändern. Somit kann er je nach Bedarf bzw. Indikation die Flächenpressung während des Einsatzes erhöhen oder verringern.

Im Konkreten wirkt der Hebel 40 gemäß der Fig. 9 ausschließlich über die eine Zug-/Druckfeder 30 auf den Schaft-Getriebezug, die bei Aufeinanderliegen der Zangenbranchen 50, 52 komprimiert/deformiert wird, wobei der Hebel 40 ab einem bestimmten Einschwenkwinkel mit dem Instrumentengriff in Anschlag kommt und somit ein weiteres Verschwenken bzw. weiteres Deformieren der Feder 30 verhindert wird. Dabei kann der Federsitz auf Seiten des Hebels 40 verstellt werden, um im Rahmen des maximalen Einschwenkwinkels eine größere oder kleinere Federdeformation (Vorspannweg) zu bewirken, wodurch die maximal erreichbare Federkraft und damit der Klemmdruck einstellbar wird.

Figur 10 zeigt ein weiteres bevorzugtes Ausführungsbeispiel eines elektrochirurgischen Instruments der vorliegenden Erfindung in Form eines schaftlosen, linearen Versiegelungsinstruments mit zwei linearen Zangenbranchen 50, 52 die distal unmittelbar in einem Instrumentengriff 4 zangenartig gelagert sind. Am Griffstück 4 ist ein Hebel oder Betätigungsbügel 40 schwenkbar angelenkt, über den die Zangenbranchen 50, 52 geöffnet und geschlossen werden können. Der Hebel 40 wirkt dabei ausschließlich über eine Druckfeder 30 auf den nachgeschalteten Betätigungszug innerhalb des Griffstücks 4 zum Verschwenken der Zangenbranchen 50, 52, wobei ein hebelseitiger Federsitz durch eine in den Hebel 40 eingedrehte Stellschraube 56 gebildet wird.

Auch in diesem Fall wird die Maximalverschwenkung des Hebels 40 durch einen Anschlag im Griffstück 4 begrenzt, wodurch die maximale Federvorspannung ab in Anlagekommen der Zangenbranchen 50, 52 entsprechend begrenzt ist. Durch Verstellen der Stellschraube 56 kann dabei der Wert der maximal erreichbaren Federvorspannung innerhalb des maximalen Einschwenkwinkels des Hebels 40 eingestellt werden.

D.h., bei dem linearen Versiegelungsinstrument gemäß der Fig. 10 kann die Druckfeder 30 im mittleren Bereich des Instruments, hier vor dem Handgriff, d.h. auf der Betätigungsseite des Hebels 40, angeordnet sein. Über die Position eines Schwenkstifts, der als Drehlager für den Hebel 40 an der Griffschale 4 wirkt, kann der maximale Einschwenkwinkel und damit die Vorspannung der Feder 30 bei bereits geschlossenen Zangenbranchen 50, 52 eingestellt werden. Dadurch wird die im geschlossenen Zustand des Instruments wirkende Flächenpressung gesteuert. Als Einstellmöglichkeit ist in dieser Ausführungsform die Einstellschraube 56 dargestellt, die die Vorspannung der Feder 30 bei geschlossenen Zangenbranchen 50, 52 einstellt.

Dabei sei darauf hingewiesen, dass Anstelle einer Stellschraube 56 auch ein anderes Einstellprinzip, beispielsweise ein Einstellkeil mit nachgeschaltetem axialverschieblichem Federsitzbolzen vorgesehen sein kann. In diesem Fall gemäß der Fig. 11 würde der Keil mittels einer Schiebetaste verschoben, an der gleichzeitig an eine Anzeigeskala beispielsweise für unterschiedliche Körpergewebe aufgebracht sein könnte.

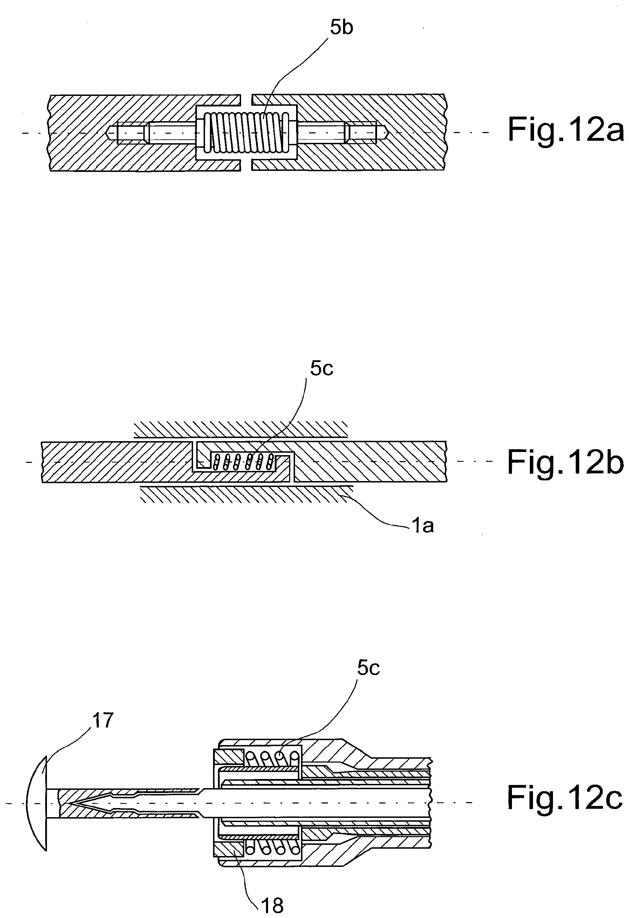

D.h., bei derartigen linearen Versiegelungsinstrumenten ist insbesondere im Hinblick auf die verschiedenen Indikationen und Gewebearten, bei denen das Instrument eingesetzt wird, die Einstellmöglichkeit der Flächenpressung wichtig. Nachfolgend werden mit Bezug auf Figuren 12A bis 12C daher einige Lösungsvorschläge für diese Einstellung weiter aufgeführt. Es ist anzumerken, dass der Fachmann derartige Lösungsansätze sowohl auf lineare als auch auf zirkuläre Versiegelungsinstrumente übertragen kann, weshalb sie hier nur allgemein an einem auf alle vorstehend erörterten Ausführungsformen übertragbaren prinzipiellen Beispiel beschrieben werden.

Statt der Druckfeder der ersten Ausführungsform kann auch eine Tellerfedersäule, die die Druckfeder direkt ersetzen kann, oder eine Zugfeder verbaut werden. Figur 6A zeigt ein Beispiel mit einer Zugfeder 5b, die zwei Teile des Versiegelungsinstruments beispielsweise die Hohlspindel und die darin gelagerte Zug-/Druck-Stange zusammenzieht. Auch kann die Druckfeder bzw. die Zugfeder gemäß der Fig. 12a irgendwo in den Kraftübertragungszug zu der einen beweglichen Elektrodenbranche zwischengefügt sein.

Figur 12B zeigt eine weitere alternative Ausführungsform mit einer Druckfeder, die irgendwo im Kraftübertragungszug auf die zumindest eine Elektrodenbranche zwischengefügt ist.

Figur 12C zeigt eine Ausführungsform, in der wie in Fig. 12B eine Druckfeder eingesetzt wird. In diesem Beispiel ist die Druckfeder jedoch im Instrumentenkopf enes zirkulären elektrochirurgischen Instruments angeordnet und wirkt somit direkt auf die nunmehr im Instrumentenkopf axialbewegbar gelagerte Instrumentenbranche als federvorgespanntes Gegenlager zum Amboss.

Durch Verstellen des Mechanismus im Wesentlichen bestehend aus Mutter, Spindel und Zug-/Druckfeder sowie der über die Feder mit der Spindel wirkverbundenen Zug-Druck-Stange kann wie in den vorstehend erläuterten Ausführungsformen gezeigt ist, insbesondere bei zirkulären Versiegelungsinstrumenten abhängig von der Gewebedicke eine Vorspannkraft/Klemmkraft aufgebracht werden. Alternativ zu den bisher beschriebenen Gewinden können auch auf andere Art entsprechende Vorspannkräfte aufgebracht werden, beispielsweise über exzentrische verstellbare Spannnoppen. Wird dünneres Gewebe eingespannt, kann beispielsweise über die Zustellung bzw. Drehung eines Exzenters, der entweder die Federvorspannung oder den Abstand zwischen den Klemmbacken beeinflusst, die Vorspannkraft und/oder die Flächenpressung im Gewebe erhöht werden. Alternativ können die bereits erörterten Schrauben zu diesem Zweck eingesetzt werden. Bei dickeren Gewebearten kann dagegen durch Zurückdrehen des Exzenters die Vorspannkraft oder Flächenpressung verringert und somit eine mechanische Gewebeschädigung in Folge der Pressung verhindert werden.

Wie vorstehend bereits ausgeführt wurde, kann die Klemmdruck-Regeleinrichtung sowohl im Instrumentengriff beispielsweise bei Schaft-Instrumenten mit distalen Instrumentenbranchen oder bei linearen zangenartigen Instrumenten (ohne Schaft) eingebaut und in den dort beginnenden Kraft-/ Moment-Übertragungszug zwischengefügt werden. Alternativ hierzu ist es prinzipiell auch möglich, die Klemmdruck-Regeleinrichtung im Bereich des distalen Instrumentenkopfs bei Schaft-Instrumenten wie beispielsweise einem Anastomose-Instrument in den in den dort beginnenden Kraft-/ Moment-Übertragungszug zu integrieren. Hierfür kann die Klemm-Regeleinrichtung individuell konstruktiv ausgestaltet sein, um sich sowohl an die räumlichen Bedingungen wie auch entsprechend der dort aktuell vorliegenden Übertragungszug-Bauteile einzufügen.

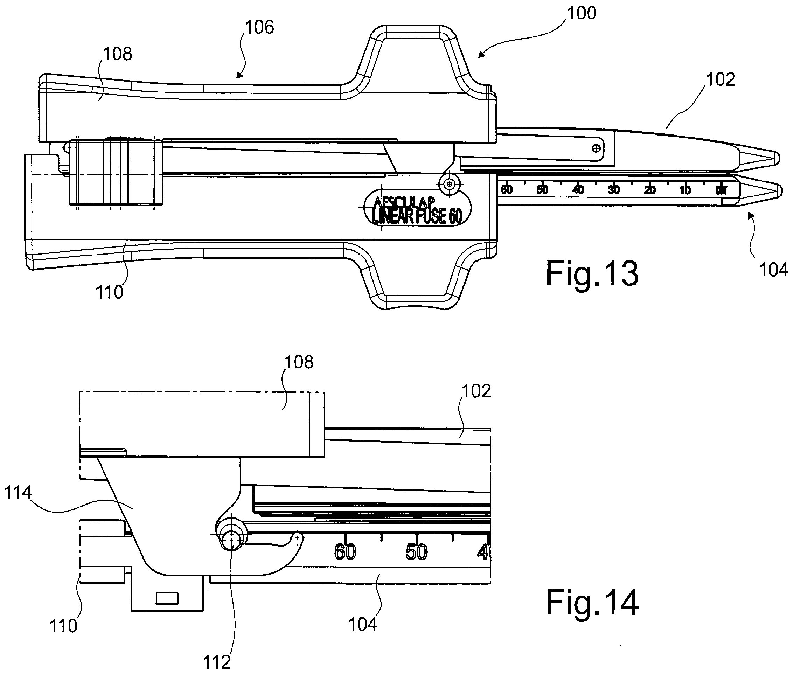

In den Fig. 13 bis 16 sind daher weitere alternative Ausgestaltungen für eine Klemmdruck-Regeleinrichtung innerhalb des Instrumentengriffs am Beispiel eines linearen Instruments 100 aufgezeigt.

So hat ein lineares elektrochirurgisches Instrument 100 in bekannter Weise zwei lineare Instrumentenbranchen 102, 104, die innerhalb eines Instrumentengriffs 106 vorzugsweise scherenartig aneinander scharniert sind und proximal zur Scharnierstelle zu zwei Griffhebeln 108, 110 verlängert sind. An einem der Griffhebel110 bzw. an einem der Instrumentenbranchen 104 ist gemäß der Fig. 13 und 14 ein Querbolzen 112 angeformt oder eingesetzt, mit welchem eine Spannklinke 114 in Spanneingriff bringbar ist, welche am anderen Griffhebel 106 angelenkt ist, um eine vorbestimmte Zusammen-Spannkraft auf die beiden Instrumentenbranchen 102, 104 beim vollständigen Einrasten/Verriegeln der Spannklinke 114 mit dem Querbolzen 112 gemäß der Fig. 14 aufzubringen. Die vorbestimmte Zusammen-Spannkraft ist demnach abhängig von der Relativlage des Querbolzens 112 zur Spannklinke 114 in eingerastetem/verriegeltem Zustand.

Dies bietet beispielsweise die Möglichkeit, die Klemmdruck-Regeleinrichtung genau in diesem Bereich des Kraft-/Moment-Übertragungszugs einzufügen.

Gemäß der Fig. 14 ist nämlich der Querbolzen 112 als ein Exzenter ausgebildet, die sich bei einem Verdrehen an die Spannklinke 114 (bzw. deren Eingriffsabschnitt) annähert oder hiervon entfernt. Somit ist es möglich, die Spannkraft zwischen den Instrumentenbranchen 102, 104 bei verriegeltem Zustand der Spannklinke 114 am Excenter-Querbolzen 112 voreinzustellen und so das Instrument an unterschiedliche Gewebe anzupassen.

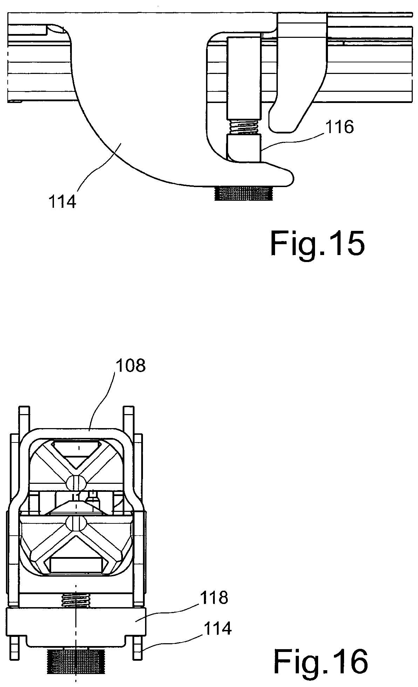

Alternativ hierzu ist es aber auch möglich, den vorstehend genannten Querbolzen 112 durch einen Spannmechanismus zu ersetzen, wie er in den Fig. 15 und 16 angedeutet ist.

In diesem Fall ist an dem einen Griffhebel 108 ebenfalls eine Spannklinke 114 montiert, an deren freiem Klinkenende ein Eigriffsabschnitt ausgeformt ist, der mit einer Zug-/Druck-Schraube 116 in Formschluss -Eingriff bringbar ist. Diese Schraube 116 ist an den anderen Griffhebel 110 bzw. an die damit verbundene Instrumentenbranche 104 gekoppelt, um darauf eine Druckkraft in Axialrichtung des Schraube 116 aufzubringen.

Die Schraube 116 hat hierfür ein Krafteinleitungsbauteil (Riegel) 118, der mit dem Eingriffsabschnitt der Spannklinke 114 in Eingriff bringbar ist, um die vorstehend genannte Zusammen-Spannkraft auf die Instrumentenbranchen 102, 104 entsprechend der Spannklinken-Position aufzubringen. Dieses Krafteinleitungsbauteil 116 ist dabei bei Drehen der Schraube 116 bezüglich der Spannklinke 114 relativ bewegbar, um so die Zusammen-Spannkraft der Instrumentenbranchen in verriegelter Spannklinkenposition (vor-) einstellen zu können.

In beiden Varianten der Klemmdruck-Regeleinrichtung ist demnach die Relativposition zwischen Spannklinke und Querbolzen/Krafteinleitungsbauteil verstellbar, wobei in diesem Fall auf eine Feder als Kraftspeicher verzichtet wird. Dabei sei jedoch ausdrücklich darauf hingewiesen, dass auch der Querbolzen 112 oder das Krafteinleitungsbauteil 118 federnd gelagert sein kann, um einen Kraftspeicher im Sinne der vorstehenden Ausführungsbeispiele zu verwirklichen.

Damit es zu keiner Leckage der Versiegelung kommt, ist es bei der vorliegenden Erfindung vorteilhaft und ggf. wichtig, die auf das Gewebe wirkenden Parameter zu erfassen und zu regeln. Neben der Flächenpressung, für deren Regelung die vorstehend beschriebenen Ausführungsformen dienen, gehören Temperatur, Druck, Gewebeimpedanz, Abstand der Elektroden und die Lage des Gewebes im Körper zu diesen Parametern.

Bei einem zirkulären Versiegelungsinstrument zur Herstellung einer End-zu-End-Anastomose wie vorstehend beschrieben, müssen mehrere sicherheitsrelevante Schritte in der richtigen Reihenfolge durchgeführt werden. Damit der Anwender diese korrekte Abfolge einhält, können zusätzlich zu den bisher erläuterten Einrichtungen zur Einstellung der Flächenpressung entsprechende Sicherheitsvorrichtungen im Instrument vorgesehen werden. Dabei sind folgende Punkte zu beachten:
Der HF-Strom darf nicht aktiviert werden können, wenn das Instrument offen ist.

Der HF-Strom darf nicht aktiviert werden können, wenn nicht der richtige Bereich der Flächenpressung eingestellt ist.

Der Schneidvorgang darf nicht freigegeben werden, bevor die HF-Versiegelung durchgeführt wurde.

Die Sicherheitsvorkehrungen an üblichen zirkulären Klammernahtinstrumenten wie vorstehend als Stand der Technik erörtert sind rein mechanisch und können bei falscher Handhabung übergangen werden. Zum Beispiel ist das Auslösen von Klammern und der Klinge ohne aufgesetzten Amboss möglich, wenn der Trokardorn ohne adaptierten Amboss komplett eingefahren ist.

Die vorliegende Ausführungsform soll geeignete Sicherheitsvorkehrungen insbesondere für ein erfindungsgemäßes zirkuläres Versiegelungsinstrument zur Verfügung stellen.

Im distalen Instrumentenkopf befindet sich dazu wie in den Figuren 5 und 6 gezeigt eine durchgängige axiale Kontaktbuchse 60, die mit der HF-Betätigungseinrichtung 18 am Instrumentengriff 4 über elektrische Leitungen Kontakt hat und durch die sich der Trokardorn 14 in axialer Richtung bewegen kann. Am Trokardorn 14 ist eine weitere Kontaktbuchse 62 fixiert, die mit den Elektroden 17 am Amboss 18 elektrisch verbunden ist. Der Trokardorn 14 selbst ist aus einem elektrisch nicht leitenden Material (zumindest abschnittsweise) gefertigt. Diese weitere Kontaktbuchse 62 ist am Trokardorn 14 so axial platziert, dass sie mit der durchgängigen Kontaktbuchse 60 nur dann elektrischen Kontakt erhält, wenn der Amboss 18 komplett in Richtung Instrumentenkopf 2 eingezogen ist. Wenn der Trokardorn 14 ausgefahren ist, besteht kein elektrisch leitender Kontakt zur durchgängigen Kontaktbuchse 60. Damit ist sichergestellt, dass kein HF-Strom aktiviert werden kann, wenn das Instrument offen ist, da sonst eine Fehlermeldung ausgegeben wird.

Im proximalen Handgriff 4, der insbesondere in Fig. 4 veranschaulicht ist, befindet sich der manuell betätigbare HF-Auslösetaster (HF-Betätigungseinrichtung) 18 und ein weiterer Kontrolltaster 64. Der Kontrolltaster 64 ist vorliegend an den Anzeigeriegel/das Anzeigeblech 32 der Flächenpressungs-Anzeigeskala wirkgekoppelt und ist nur dann (vom Riegel) betätigt, wenn der richtige Bereich der Flächenpressung eingestellt ist. Wird der Bereich der korrekten Flächenpressung überschritten oder unterschritten, ist der Kontrolltaster 32 nicht betätigt. Somit wird sichergestellt, dass es zu keiner Aktivierung der HF-Stromzufuhr über den HF-Auslösetaster 18 kommt, bei der der falsche (oder kein) Klemmdruck eingestellt ist. Weiterhin ist es denkbar, den richtigen Bereich der Flächenpressung mit Farben, z.B. rot und grün, oder auf ähnliche Weise optisch hervorzuheben.

Nach dem korrekten Auslösen des HF-Stroms wird ein Hubmagnet 66 (elektrisch) aktiviert bzw. eingezogen, an dem ein Sperrstift 68 angeordnet ist, der in die Bewegungsmechanik des Schneidklingen-Betätigungshebels 40 eingreift und diesen im ausgefahrenen Zustand blockiert. Im eingezogenen Zustand gibt der Sperrstift 68 den Schneidvorgang nunmehr jedoch frei, wenn die Versiegelung durch den HF-Strom abgeschlossen ist. Bei einer fehlerhaften Bestromung (Versagen der automatischen Ansteuerung des Hubmagneten) besteht optional die Möglichkeit, den Schneidvorgang durch eine entsprechende Eingabe am (nicht gezeigten) Generator freizugeben.

Eine weitere (nicht in den Figuren dargestellte) Sicherheitseinrichtung sieht eine mechanische Notfallbetätigung für die Schneidklinge vor, welche den Hubmagneten entweder brückt oder dessen Hubkraft überwindet. Damit sind Generator- und Stromausfälle abgesichert. Die mechanische Notfallbetätigung muss deutlich sichtbar am Instrument befestigt sein und darf nicht ungewollt betätigt werden können. Dies kann z.B. durch einer Art Entriegelungsraste oder eine zu zerstörende / bewusst zu entfernende Abdeckung realisiert werden.

Der Kontrolltaster 64 für die Abfrage/Detektion der richtigen Flächenpressung, der mit der korrekten Einstellung der Flächenpressung wie vorstehend beschrieben gekoppelt ist, stellt eine neuartige Sicherheitsvorkehrung für zirkuläre Klammernahtinstrumente und/oder zirkuläre Versiegelungsinstrumente aber auch für Linearinstrumente dar. In Kombination mit dem Hubmagnet 68 und den Kontakten im Instrumentenkopf 2 wie in der vorstehend erörterten Ausführungsform beschrieben bietet dieses Konzept dem Anwender größere Sicherheit als ein (derzeit bekanntes) Klammernahtinstrument. Dadurch ergibt sich maximale Kontrolle und Sicherheit für den Anwender und den Patienten im Bezug auf die folgenden drei relevanten Punkte:
- Es wird abgefragt, ob das Instrument geschlossen ist.
- Es wird abgefragt, ob die richtige Flächenpressung eingestellt wurde.
- Es kann nicht geschnitten werden, bevor nicht versiegelt wurde.

Statt der Kontaktbuchsen können jegliche elektrische Kontakte verwendet werden, die den gleichen Zweck erfüllen. Ebenso verhält es sich bei dem HF-Auslösetaster und dem Kontrolltaster. Der Hubmagnet kann durch andere elektromechanische Verriegelungselemente ersetzt werden.

Obwohl die Sicherung in den Vorliegenden Ausführungsformen am Beispiel von Instrumenten für die HF-Versiegelung gezeigt ist, können vergleichbare Vorrichtungen zur Sicherstellung, dass erst nach dem Versiegeln geschnitten wird, prinzipiell auch bei Klammernahtinstrumenten oder anderen Instrumenten zur Herstellung von Anastomosen verwendet werden.

Die vorliegende Erfindung offenbart zusammenfassend Mechaniken für die Einstellung einer Flächenpressung insbesondere für Instrumente zur Gewebeversiegelung bei Anastomosen. Die Einstellbarkeit der Flächenpressung wie in der Erfindung beschrieben kann auch für Klammernahtinstrumente genutzt werden. Besonders bevorzugt werden zusätzliche Sicherungseinrichtungen vorgesehen, die das Auslösen der Anastomose verhindern, wenn sich das Instrument nicht in einem gesicherten Zustand befindet, in dem die Anastomose ausgelöst werden soll.

## Patentansprüche

1. Chirurgisches Instrument vorzugsweise der HF-Bauart mit zwei Gewebebranchen (2, 18, 50, 52), von denen zumindest eine relativ zur anderen bewegbar ist und die über einen Betätigungsmechanismus (20, 40) unter dazwischen Einklemmen von Körpergewebe mit einem vorbestimmten oder vorbestimmbaren Anpressdruck an die andere Gewebebranche anlegbar ist, und mit einer Klemmdruckregel- oder -einstelleinrichtung (22, 30), die in einem Kraft- oder Momentübertragungszug zwischen dem Betätigungsmechanismus (20, 40) und der zumindest einen bewegbaren Gewebebranche oder innerhalb des Betätigungsmechanismus (20, 40) zwischengeschaltet ist und zumindest zwei gegeneinander axial verschiebbare Schub-Zugelemente (22, 24) hat, zwischen denen ein Kraftspeicher in Form einer axial wirkenden Druckfeder (30) für eine elastische Kraftübertragung zwischengefügt ist, **dadurch gekennzeichnet, dass** die unbetätigte, für die Druckfedervorspannung relevante Relativlage zwischen den zumindest zwei Schub-Zugelementen (22, 24) zur Vorbestimmung eines maximal erreichbaren Anpressdrucks einstellbar ist.

2. Chirurgisches Instrument nach Anspruch 1, **dadurch gekennzeichnet, dass** die Klemmdruckregel- oder -einstelleinrichtung (22, 30) nach Art eines elastisch nachgebenden Hubzylinders ausgebildet ist, dessen elastische Nachgiebigkeit in Axialrichtung vorzugsweise einstellbar ist.

3. Chirurgisches Instrument nach Anspruch 1 oder 2, **dadurch gekennzeichnet, dass** der Kraftspeicher durch den Betätigungsmechanismus (20, 40) um einen definierten/definierbaren Betrag oder innerhalb eines definierten/definierbaren Betragsbereichs aufladbar ist, bei welchem eine/ein daraus resultierende(s) sowie über den Kraft- oder Momentübertragungszug übertragene(s) Betätigungskraft oder Betätigungsmoment den vorbestimmten oder vorbestimmbaren Anpressdruck erzeugt.

4. Chirurgisches Instrument nach Anspruch 3, **dadurch gekennzeichnet, dass** die axial wirkende Druckfeder Betätigungsbetrags-entsprechend deformierbar ist.

5. Chirurgisches Instrument nach Anspruch 4, **dadurch gekennzeichnet, dass** die gegeneinander bewegbaren Schub-Zuglemente (22, 24) mit Federsitzen (26, 28) ausgebildet sind, von denen zumindest einer verstellbar ist, um im Rahmen eines maximal vorgesehenen Betätigungswegs oder Betätigungsbetrags des Betätigungsmechanismus unterschiedliche Deformationsbeträge der Feder (30) zu bewirken.

6. Chirurgisches Instrument nach einem der vorstehenden Ansprüche 4 oder 5, **dadurch gekennzeichnet, dass** der Betätigungsmechanismus ein Handhebel, ein manuell betätigbarer Drehknopf oder ein motorischer Antrieb ist, der mit einem ersten Schub-Zugelement der Klemmdruckregel- oder -einstelleinrichtung für dessen Axialverschiebung direkt in Wirkeingriff steht.

7. Chirurgisches Instrument nach Anspruch 6, **dadurch gekennzeichnet, dass** im Fall eines Drehknopfs dieser an einem Instrumentengehäuse gelagert ist und über ein Innengewinde mit einem hülsenförmigen Spindelkörper (20) als das erstes Schub-Zugelement für dessen Axialbewegung in Eingriff ist, das über die Druckfeder (30) mit einem darin axial verschiebbar gelagerten Schaft (24) als das zweiten Schub-Zugelement in Axialanlage steht.

8. Chirurgisches Instrument nach einem der vorstehenden Ansprüche insbesondere nach Anspruch 6, **dadurch gekennzeichnet, dass** der Kraftspeicher oder das erste Schub-Zugelement (20) mit einer Anzeigeeinrichtung (32) vorzugsweise mechanisch gekoppelt ist, über die der durch den Betätigungsmechanismus erwirkte Ladebetrag detektier- und vorzugsweise visuell als aktueller Branchen-Anpressdruck anzeigbar ist.

9. Chirurgisches Instrument nach Anspruch 8 in Verbindung mit Anspruch 5, **dadurch gekennzeichnet, dass** die Anzeigeeinrichtung ein mit einem der Federsitze (26) wirkgekoppelter Mitnahmeriegel (32) ist, der mit Beginn einer Federdeformation von dem Federsitz mitgenommen wird und dadurch den Deformationsweg abgreift und vorzugsweise anzeigt.

10. Chirurgisches Instrument nach einem der vorstehenden Ansprüche, **gekennzeichnet durch** einen Anschlag zur Begrenzung des maximalen Betätigungswegs des Betätigungsmechanismus.

11. Chirurgisches Instrument nach einem der vorstehenden Ansprüche, **gekennzeichnet durch** ein Kraft- oder Drehmoment-Begrenzungsmittel vorzugsweise in Form einer Rutschkupplung, das zwischen dem Betätigungsmechanismus und dem Kraftspeicher angeordnet ist.

## Claims

1. A surgical instrument preferably in HF design, comprising two tissue branches (2, 18, 50, 52), at least one of them being movable relative to the other one and being able to be applied to the other tissue branch with a predetermined or predeterminable contact pressure via an actuation mechanism (20, 40) while clamping body tissue therebetween, and with a clamping pressure controlling or adjustment device (22, 30) which is interconnected in a force or torque gear train between the actuation mechanism (20, 40) and the at least one movable tissue branch or within the actuation mechanism (20, 40) and which has at least two push/pull elements (22, 24) which can be axially shifted relative to each other, an energy accumulator in the form of an axially acting compression spring (30) being provided between said push/pull elements for the purpose of an elastic force transmission, **characterized in that** the non-actuated relative position, which is relevant for the preload of the compression spring, between the at least two push/pull elements (22, 24) is able to be adjusted for predetermining the maximum attainable contact pressure.

2. The surgical instrument according to claim 1, **characterized in that** the clamping pressure controlling or adjustment device (22, 30) is designed in the nature of an elastically yielding lifting cylinder whose elastic resilience can be adjusted preferably in the axial direction.

3. The surgical instrument according to claim 1 or 2, **characterized in that** the energy accumulator can be loaded through the actuation mechanism (20, 40) by a defined/definable amount or can be loaded within a defined/definable range of the amount, in which an actuation force or actuation moment resulting therefrom and transmitted by the force or torque gear train produces the predetermined or predeterminable contact pressure.

4. The surgical instrument according to claim 3, **characterized in that** the axially acting compression spring can be deformed according to the actuation amount.

5. The surgical instrument according to claim 4, **characterized in that** the push/pull elements (22, 24) which can be moved relative to each other are provided with spring seats (26, 28), at least one of which is able to be adjusted in order to produce different deformation amounts of the spring (30) within the maximum actuating travel or actuation amount of the actuation mechanism.

6. The surgical instrument according to any one of the preceding claims 4 or 5, **characterized in that** the actuation mechanism is a manual lever, a manually operable rotary knob or a motor-assisted drive system, which is in direct operative connection with a first push/pull element of the clamping pressure controlling or adjustment device for the axial displacement thereof.

7. The surgical instrument according to claim 6, **characterized in that** if there is a rotary knob, the latter is supported on an instrument case and, via an internal thread, is in engagement with a sleeve-shaped spindle body (20) as the first push/pull element for its axial movement, said first push/pull element being in axial contact via the compression spring (30) with a shaft (24) being axially shiftable therein and representing the second push/pull element.

8. The surgical instrument according to any one of the preceding claims, in particular according to claim 6, **characterized in that** the energy accumulator or the first push/pull element (20) is preferably mechanically coupled to a display device (32) which is able to detect the loading amount caused by the actuation mechanism and to display it as a current branch contact pressure preferably in a visual manner.

9. The surgical instrument according to claim 8 in combination with claim 5, **characterized in that** the display device is a drive bar (32) which is operatively coupled to one of the spring seats (26) and is entrained by the spring seat when the spring begins to deform, thus calipering the deformation path and preferably displaying it.

10. The surgical instrument according to any one of the preceding claims, **characterized by** a stop for limiting the maximum actuating travel of the actuation mechanism.

11. The surgical instrument according to any one of the preceding claims, **characterized by a** force or torque limiting means preferably in the form of a sliding clutch which is arranged between the actuation mechanism and the energy accumulator.

## Revendications

1. Instrument chirurgical de préférence de type HF avec deux branches tissulaires (2, 18, 50, 52), dont au moins une est mobile par rapport à l'autre et qui peut être appliquée contre l'autre branche tissulaire par l'intermédiaire d'un mécanisme d'actionnement (20, 40) en pinçant le tissu corporel entre elles avec une pression d'appui prédéfinie ou prédéfinissable, et avec un dispositif de réglage ou de régulation de la pression de pincement (22, 30), qui est interconnecté dans un train de transmission de force ou de couple entre le mécanisme d'actionnement (20, 40) et l'au moins une branche tissulaire mobile ou à l'intérieur du mécanisme d'actionnement (20, 40) et a au moins deux éléments de poussée-traction (22, 24) pouvant être déplacés axialement l'un par rapport à l'autre, entre lesquels un accumulateur de force est interposé sous la forme d'un ressort de pression à action axiale (30) pour une transmission de force élastique, **caractérisé en ce que** la position relative non actionnée, pertinente pour la précontrainte du ressort de pression est réglable entre les au moins deux éléments de poussée-traction (22, 24) pour la prédétermination d'une pression d'appui maximum réalisable.

2. Instrument chirurgical selon la revendication 1, **caractérisé en ce que** le dispositif de réglage ou de régulation de la pression de pincement (22, 30) est réalisé à la manière d'un vérin de levage fléchissant élastiquement, dont le fléchissement élastique est réglable de préférence dans la direction axiale.

3. Instrument chirurgical selon la revendication 1 ou 2, **caractérisé en ce que** l'accumulateur de pression peut être chargé par le mécanisme d'actionnement (20, 40) d'une valeur définie/définissable ou à l'intérieur d'une plage de valeurs définie/définissable, dans laquelle une force d'actionnement ou un couple d'actionnement en résultant et transmis(e) par l'intermédiaire du train de transmission de force ou de couple génère la pression d'appui prédéfinie ou prédéfinissable.

4. Instrument chirurgical selon la revendication 3, **caractérisé en ce que** le ressort de pression à action axiale est déformable selon une valeur d'actionnement.

5. Instrument chirurgical selon la revendication 4, **caractérisé en ce que** les éléments de poussée-traction (22, 24) mobiles l'un par rapport à l'autre sont réalisés avec des sièges de ressort (26, 28), dont au moins un est réglable, pour entraîner dans le cadre d'une course d'actionnement ou d'une valeur d'actionnement maximum prévue du mécanisme d'actionnement des valeurs de déformation différentes du ressort (30).

6. Instrument chirurgical selon l'une quelconque des revendications précédentes 4 ou 5, **caractérisé en ce que** le mécanisme d'actionnement est un levier manuel, un bouton rotatif manuellement actionnable ou un entraînement moteur, qui est directement en prise fonctionnelle avec un premier élément de poussée-traction du dispositif de réglage ou de régulation de la pression de pincement pour son déplacement axial.

7. Instrument chirurgical selon la revendication 6, **caractérisé en ce que** dans le cas d'un bouton rotatif, celui-ci est logé au niveau d'un boîtier d'instrument et est en prise par l'intermédiaire d'un filetage intérieur avec un corps de broche en forme de douille (20) en tant que premier élément de poussée-traction pour son déplacement axial, qui est en appui axial par le biais du ressort de pression (30) avec un arbre (24) logé de manière axialement mobile en tant que deuxième élément de poussée-traction.

8. Instrument chirurgical selon l'une quelconque des revendications précédentes en particulier selon la revendication 6, **caractérisé en ce que** l'accumulateur de force ou le premier élément de poussée-traction (20) est couplé de préférence mécaniquement à un dispositif d'affichage (32), par l'intermédiaire duquel la valeur de charge obtenue par le mécanisme d'actionnement peut être détectée et de préférence affichée visuellement en tant que pression d'appui de branche.

9. Instrument chirurgical selon la revendication 8 en relation avec la revendication 5, **caractérisé en ce que** le dispositif d'affichage est un verrou d'entraînement (32) couplé fonctionnellement à un des sièges de ressort (26), qui est entraîné au début de la déformation de ressort par le siège de ressort et détecte ainsi et affiche de préférence la course de déformation.

10. Instrument chirurgical selon l'une quelconque des revendications précédentes, **caractérisé par** une butée de limitation de la course d'actionnement maximum du mécanisme d'actionnement.

11. Instrument chirurgical selon l'une quelconque des revendications précédentes, **caractérisé par** un moyen de limitation de force ou de couple, de préférence sous la forme d'un accouplement glissant, qui est agencé entre le mécanisme d'actionnement et l'accumulateur de force.
